# EUROPEAN PATENT SPECIFICATION

(11) **EP 4 162 943 B1**
(45) Date of publication and mention of the grant of the patent: **10.04.2024**
(21) Application number: 22199921.2
(22) Date of filing: 06.10.2022
(51) Int. Cl.: A61K 36/185, A61K 36/889, A61K 36/32, A61P 15/00, A61P 5/28, A61K 31/355, A61K 33/04, A61K 36/736, A61K 45/06, A61K 33/30

(54) **COMPOSITION FOR THE TREATMENT AND THE PREVENTION OF PATHOLOGIES OF THE PROSTATE AND URINARY TRACT**
ZUSAMMENSETZUNG ZUR BEHANDLUNG UND PRÄVENTION VON ERKRANKUNGEN DER PROSTATA UND DER HARNWEGE
COMPOSITION POUR LE TRAITEMENT ET LA PRÉVENTION DE PATHOLOGIES DE LA PROSTATE ET DES VOIES URINAIRES

(30) Priority: 07.10.2021 IT 202100025652
(43) Date of publication of application: 12.04.2023
(73) Proprietor: Anvest Group S.r.l., 20152 Milano (IT)
(72) Inventor: ALFIERI, Sandro, 84083 Castel San Giorgio (IT)
(74) Representative: Cattaneo, Elisabetta

(56) References cited:
- WO-A1-2017/108907
- ALLKANJARI OLTA ET AL: "What do we know about phytotherapy of benign prostatic hyperplasia?", LIFE SCIENCE, PERGAMON PRESS, OXFORD, GB, vol. 126, 20 February 2015 (2015-02-20), pages 42-56, XP029146242, ISSN: 0024-3205, DOI: 10.1016/J.LFS.2015.01.023
- AZIMI HANIEH ET AL: "A Review of Animal and Human Studies for Management of Benign Prostatic Hyperplasia with Natural Products: Perspective of New Pharmacological Agents", INFLAMMATION & ALLERGY DRUG TARGETS, BENTHAM SCIENCE PUBLISHERS, NL , vol. 11, no. 3 31 May 2012 (2012-05-31), pages 207-221, XP009529299, ISSN: 1871-5281, DOI: 10.2174/187152812800392715 Retrieved from the Internet: URL:http://eurekaselect.com/openurl/conten t.php?issue=3&genre=article&volume=11&issn =1871-5281&spage=207
- ESPOSITO CRISTINA ET AL: "Epilobium angustifolium L. extract with high content in oenothein B on benign prostatic hyperplasia: A monocentric, randomized, double-blind, placebo-controlled clinical trial", BIOMEDICINE & PHARMACOTHERAPY, ELSEVIER, FR, vol. 138, 23 March 2021 (2021-03-23), XP086538637, ISSN: 0753-3322, DOI: 10.1016/J.BIOPHA.2021.111414 [retrieved on 2021-03-23]
- Aryeh Keehn ET AL: "Complementary and alternative medications for benign prostatic hyperplasia", The Canadian journal of urology, 1 October 2015 (2015-10-01), page 18, XP055592096, Canada Retrieved from the Internet: URL:https://www.canjurol.com/html/free-art icles/JUV22I5S1F_08_DrLowe.pdf
- STEENKAMP V: "Phytomedicines for the prostate", FITOTERAPIA, IDB HOLDING, MILAN, IT, vol. 74, no. 6, 1 September 2003 (2003-09-01), pages 545-552, XP009505471, ISSN: 0367-326X, DOI: 10.1016/S0367-326X(03)00155-2 [retrieved on 2003-08-02]
- CICERO ARRIGO F.G. ET AL: "Nutraceutical treatment and prevention of benign prostatic hyperplasia and prostate cancer", ARCHIVIO ITALIANO DI UROLOGIA, ANDROLOGIA : UROLOGICAL AND ANDROLOGICAL SCIENCES ; ORGANO UFFICIALE, vol. 91, no. 3, 2 October 2019 (2019-10-02), XP055914754, IT ISSN: 1124-3562, DOI: 10.4081/aiua.2019.3.139
- HOLANDA PINTO S. A. ET AL: "Anti-inflammatory effect of [alpha], [beta]-Amyrin, a pentacyclic triterpene from Protium heptaphyllum in rat model of acute periodontitis", INFLAMMOPHARMACOLOGY , vol. 16, no. 1 1 February 2008 (2008-02-01), pages 48-52, XP055914711, NL ISSN: 0925-4692, DOI: 10.1007/s10787-007-1609-x Retrieved from the Internet: URL:http://link.springer.com/article/10.10 07/s10787-007-1609-x/fulltext.html
- OLIVEIRA F: "Gastroprotective and anti-inflammatory effects of resin from Protium heptaphyllum in mice and rats", PHARMACOLOGICAL RESEARCH , vol. 49, no. 2 1 February 2004 (2004-02-01), pages 105-111, XP055914702, AMSTERDAM, NL ISSN: 1043-6618, DOI: 10.1016/j.phrs.2003.09.001 Retrieved from the Internet: URL:https://www.sciencedirect.com/science/ article/pii/S1043661803002767/pdfft?md5=1a 91929fee93821512b4aba4661c356e&pid=1-s2.0- S1043661803002767-main.pdf
- CAROLINE M MELO ET AL: "Anti-inflammatory effect of alpha,beta-amyrin, a triterpene from, on cerulein-induced acute pancreatitis in mice", INFLAMMATION RESEARCH ; OFFICIAL JOURNAL OF: THE INTERNATIONAL ASSOCIATION OF INFLAMMATION SOCIETIES THE EUROPEAN HISTAMINE RESEARCH SOCIETY, BIRKHÄUSER-VERLAG, BA, vol. 60, no. 7, 12 March 2011 (2011-03-12) , pages 673-681, XP019916957, ISSN: 1420-908X, DOI: 10.1007/S00011-011-0321-X

## Description

### FIELD OF THE INVENTION

The present invention relates to a composition comprising extracts of natural ingredients for the treatment and prevention of benign prostatic hyperplasia.

### STATE OF THE ART

The prostate is a glandular organ located under the bladder that surrounds the urethra only in male individuals. It has the function of secreting seminal fluid, which nourishes spermatozoa, transports the suspended sex cells, and controls the sperm pH, as opposed to the acidic vaginal environment, where it will be housed. Under normal conditions, its shape resembles a chestnut.

The prostate is covered by a sphincter muscle, which, based on its contraction and relaxation, regulates the passage of urine and sperm. The prostate is composed of a muscular part and a glandular component. The prostate may be subject to disorders and diseases ranging from benign enlargement to inflammatory diseases (prostatitis), to oncological diseases. Due to its location, prostate disorders affect the excretory system. The prostate produces prostate fluid, which is the major nourishment for spermatozoa, consisting of a milky, slightly acidic fluid (pH 6.5) that contains numerous enzymes, zinc and citric acid that stimulate sperm motility. During ejaculation, the peristaltic contractions of the muscle fibers compress the prostate, which releases secretions into the prostatic urethra. In the prostate fluid, PSA (prostate specific antigen) has the function of thinning the spermatic clot, to facilitate the spermatozoa movement. In addition, PSA inhibits the coagulation of seminal fluid, so as to increase sperm motility.

Prostate growth and functions are regulated by androgens:
- Testosterone - steroid hormone produced in the testes by Leydig cells in men (and to a lesser extent by ovaries in women), as well as by the adrenal cortex. It is responsible for the development of secondary sexual characteristics and a possible deficit thereof leads to a decrease in libido and fertility.
- Dihydrotestosterone - active form of testosterone, synthesized mainly in the prostate and testes, through conversion by the 5α-reductase enzyme.

Benign prostatic hypertrophy (BPH), or prostate adenoma, represents the increase in volume of the prostate transition zone (the area covering the prostatic urethra), associated with a typical symptomatology of the disease, such as irritative and/or obstructive lower urinary tract symptoms (LUTS). These symptoms relating to the bladder filling and emptying phases and the post-voiding period significantly interfere with the patients' quality of life. Benign prostatic hyperplasia is the most frequent pathology in the male population over the age of 60. The underlying mechanism of this pathological condition is not yet completely clear, despite the incidence of 5-10% in forty-year-olds to around 80% in men aged 70-80. Androgens and age play an important role in the development of BPH. Local or systemic inflammation may be a factor involved in the development of BPH. In fact, several studies have shown that BPH has the characteristics of an inflammatory immune disease, and that chronic prostatic inflammation may play a role in the pathogenesis of this disease. Although the etiology of BPH is not yet fully known, an increase in the activity of aromatase, the enzyme responsible for the conversion of testosterone (TE) into estrogens, an increase in the expression of receptors for androgens and estrogens, and the presence of androgen-dependent prostate cells capable of proliferating were highlighted in hypertrophic prostate tissue. This would help explain the onset of BPH in a period of life in which plasma and tissue levels of male hormones tends to decrease. Clinical manifestations of BPH include: LUTS, incomplete bladder emptying and urinary retention, detrusor instability, urinary infection, hematuria, and eventually renal failure. Prostate cells convert testosterone (TE) into its active form, dihydrotestosterone (DHT) by means of 5 alpha reductase. DHT is the androgen responsible for stimulating protein synthesis, cell differentiation and prostate growth. While TE levels decrease with age, DHT accumulates in the prostate, binds to the androgen receptor, and activates the transcription and translation of specific DNA segments, encoding growth factors that are processed by the stroma and are responsible for modulating the proliferative and secretory activity of both the stromal portion and the epithelial portion, leading to proliferation of prostate cells which leads to BPH. Another factor responsible for the balance between proliferative processes and programmed cell death, or apoptosis, is TGF-b. BPH seems to be determined precisely by an imbalance between these two processes mediated by the various factors transcribed upon DHT stimulation.

Prostatic hyperplasia increases urethral resistance causing compensatory changes in bladder function. The increase in detrusor pressure, necessary to maintain urinary flow in the presence of greater resistance to flow, occurs at the expense of the normal filling function of the bladder. Changes in detrusor function, aggravated by anatomical changes in the bladder and nervous system related to aging, cause an increase in urinary frequency, urgency and nocturia. Current scientific evidence suggests that the bladder response to obstruction is adaptive. Most of the LUTS in patients with BPH are due to pathophysiological changes affecting the bladder rather than the obstruction itself. It has been shown that the most noticeable detrusor modification, trabeculation, is due to an increase in the collagen component in the detrusor. When trabeculation is severe, it is typically associated with significant post-voiding residue, suggesting that incomplete bladder emptying may be secondary to increased collagen component rather than impaired muscle function. Severe trabeculation is typical of advanced disease states. There are alterations in the expression of contractile muscle proteins, reduced energy production due to mitochondrial dysfunction, abnormal calcium signals and reduced intercellular communication. Although BPH is a histological definition, it is commonly associated with a clinical picture consisting of LUTS, enlarged prostate volume, chronic inflammation, and bladder obstruction.

Prostatitis usually refers to inflammation/infection of the prostate which usually involves young patients, causing symptoms and disorders very similar to those caused by prostatic hypertrophy such as pollakiuria, dysuria and difficulty in the bladder emptying phase. This is because inflammation induces edema of the prostate gland, causing enlargement with consequent compression on the urethra. The acute picture of prostatitis may be accompanied by fever. In addition to microbial infection, other causes of prostatitis are alteration of the bowel movements with irregular evacuation (diarrhea or constipation), which cause congestion of the pelvic floor and inflammation of the prostate; unbalanced diet (alcohol and spirits); sedentary lifestyle, with lack of pelvic-perineal muscles stress and subsequent "compressive" phenomena on the prostate. Prostatitis is responsible for acute prostatic symptoms, and if not treated adequately (for example in the case of therapies aimed exclusively at controlling prostatic cell growth) it can significantly delay healing. It should also be considered that prostate inflammation is accompanied in the vast majority of cases by edema. This results in an enlarged prostate causing further pain and pressure on the urinary tract with difficulty in urinating. Prostatitis may be classified as bacterial or abacterial depending on the presence or absence of a pathogen.

Furthermore, it can be:
Acute: with pain, repeated, painful and difficult urination.
Chronic: Caused by the persistence of prostate bacterial infection. It causes a sense of heaviness in the affected area, pain in the testes, difficult urination.
Asymptomatic: Difficult to diagnose since the patient does not complain of any symptoms. However, there is inflammation, which may affect the quality of prostate fluid.

The medical approach to BPH essentially aims at reducing urinary symptoms by improving the patient's quality of life, trying to significantly slow down the disease evolution. Drugs usually used, often in combination, are α-blocker or α-lytic drugs such as prazosin, doxazosin, terazosin, alfuzosin, tamsulosin, silodosin. They were born as drugs against arterial hypertension, due to their ability to decrease artery muscle contractions, and are today used mainly in prostatic hypertrophy.

The limitation of α-lytic therapy is represented by the frequent presence of side effects. These drugs have a generalized relaxing action on smooth muscle, which can cause sudden drops in blood pressure (hypotension), vertigo, dizziness, and a drop in libido. In some cases, the ejaculate may return to the bladder (retrograde ejaculation).

5α-reductase inhibitor drugs, such as Finasteride and Dutasteride, inhibit the conversion of Testosterone into Dihydrotestosterone, allowing for partial reduction in prostate volume over time. The most common side effects are decreased libido, decreased semen volume, impotence, and approximately 50% decrease in serum PSA levels. Cases of gynecomastia (increased breast volume in men) due to the accumulation of testosterone, which is converted into estrogen by the aromatase enzymes, were also reported. Recent studies have shown a synergistic therapeutic action using 5 α-reductase and α-lytic inhibitors in combination, which however also causes an enhancement of adverse events affecting the male sexual sphere, which added to the physiological reduction of libido related to age, results in devastating psychological effects within the context of a couple relationship.

The article Allkanjari Olta et Al. "What do we know about phytotherapy of benign prostatic hyperplasia?", Life science, Pergamon press, Oxford, GB, vol. 126, 20 February 2015, pages 42-56*,* discloses treatment of benign prostatic hyperplasia using herbal medicines such as combinations of extracts of *Serenoa repens, Pygeum africanum, Urtica dioica* and/or *Epilobium.* Further therapies disclosed include 5 α-reductase inhibitors or α adrenergic antagonists.

The aim of the present invention is therefore to effectively treat benign prostatic hyperplasia overcoming the adverse effects of the prior art.

### SUMMARY OF THE INVENTION

The inventors have found a combination of active ingredients that resulted to be able to effectively treat benign prostatic hyperplasia. In particular, the inventors have identified a combination of principles, some of which already known for the treatment of BPH, that combined produce a greater synergistic effect than the additive combination of known principles as will be apparent from the experimental part.

The invention therefore concerns a composition comprising an extract of *Serenoa repens,* an extract of *Pygeum africanum,* an extract of *Urtica dioica,* an extract of Epilobium and an extract of *Protium heptaphyllum.* All active ingredients are in the form of extracts as detailed below.

According to the invention, said principles may also be provided in combination with lycopene, selenium and zinc.

The invention further concerns the composition comprising an extract of *Serenoa repens,* an extract of *Pygeum africanum,* an extract of *Urtica dioica,* an extract of Epilobium and an extract of *Protium heptaphyllum* for use as a medicament.

According to a further aspect, the invention concerns the composition comprising an extract of *Serenoa repens,* an extract of *Pygeum africanum,* an extract of *Urtica dioica,* an extract of Epilobium and an extract of *Protium heptaphyllum* for use in the treatment and prevention of benign prostatic hyperplasia.

The invention therefore also concerns a supplement comprising the composition of the invention and excipients for use in the treatment of benign prostatic hyperplasia.

Advantageous aspects of the composition and supplement of the invention, such as amounts, posology and dosages will be indicated in detail in the detailed description, and the surprising synergistic effects deriving from them will be apparent in the subsequent experimental part.

In an advantageous aspect of the invention, the composition of the invention may be combined with α-lytic drugs and 5-α-reductase enzyme inhibitor drugs for the treatment of benign prostatic hyperplasia.

The composition of the invention may in fact be administered alone or in combination, *i.e.* as a co-therapeutic agent in a fixed-dose combination or in a combined therapy of separately formulated active ingredients, with at least one 5-α reductase enzyme inhibitor drug, such as for example Finasteride, Dutasteride, and/or at least one alpha-lytic drug, such as for example Doxazosin, Prazosin, Alfuzosin, Terazosin, Tamsulosin, Silodosin, for further potentiation and faster resolution of lower urinary tract symptoms (LUTS).

The composition, or the supplement comprising the same, of the invention alone or advantageously in combination with at least one antibiotic has resulted to be effective in the treatment of benign prostatic hyperplasia, in particular with regard to the symptoms associated therewith.

In fact, the composition of the invention, or the medicament or the supplement comprising the same, has an excellent anti-microbial, anti-inflammatory, and anti-androgenic activity. Specifically, the product of the invention showed excellent antimicrobial activities against Escherichia coli, anti-inflammatory activity against pro-inflammatory cytokines and/or chemokines IL-6, TNFα, and antiandrogenic activity thanks to the ability to inhibit 5-α-reductase evaluated as a reduction in the amount of Dihydrotestosterone (DHT).

### DESCRIPTION OF THE FIGURES

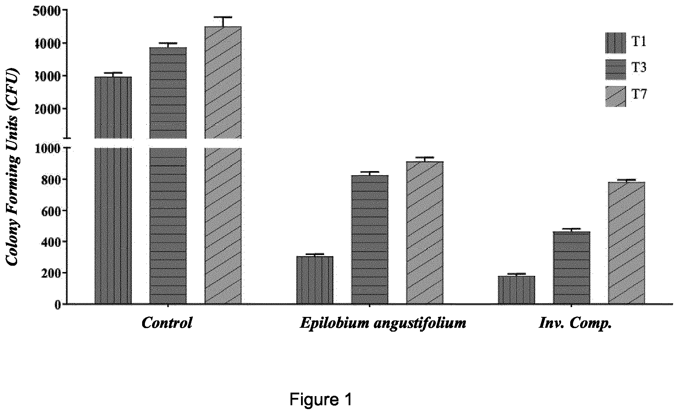

Figure 1 shows the antimicrobial activity against Escherichia coli obtained by time-killing test at 1,3,7 days of incubation of a solution containing an extract of *Epilobium angustifolium,* a solution containing the composition of the invention, and a saline solution used as a control. CFU recovered = number of E. coli colony forming units. Mean ± SD; n=6.

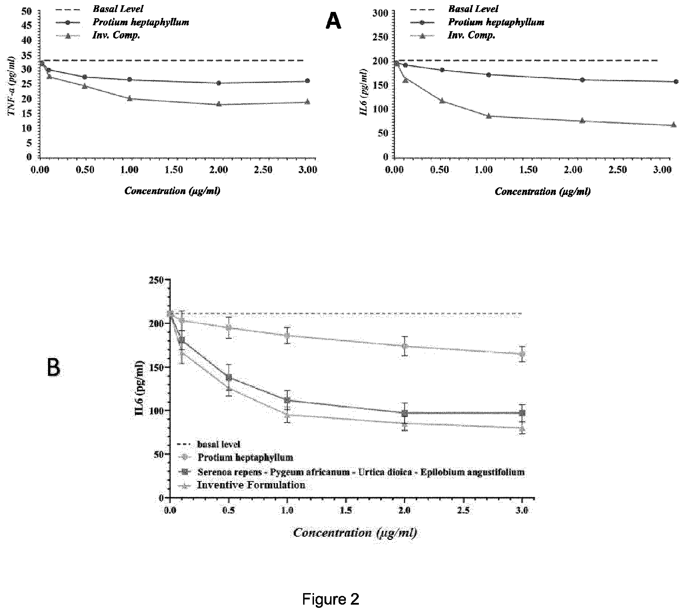

Figures 2A and 2B show IL-6 and TNFα levels after treatment of VCaP cells with extract of *Protium heptaphyllum* alone, with a composition including *Serenoa repens, Pygeum africanum, Urtica dioica,* and Epilobium as well as with the composition of the invention. The dashed line represents the basal levels. The concentrations of the single products are shown on the abscissa, the quantitative value of the marker in question expressed in pg/ml is shown on the ordinates.

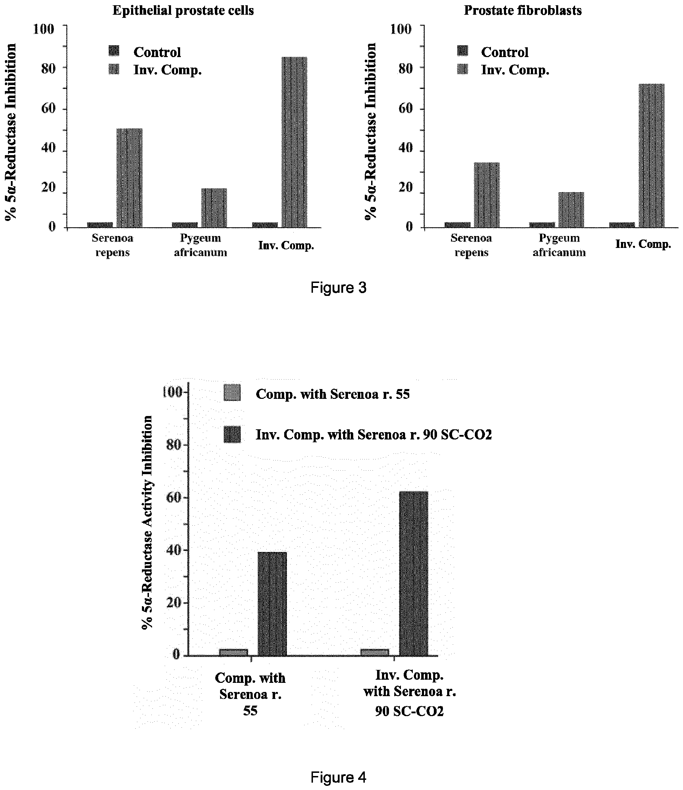

Figure 3 shows the effect of treatment with *Serenoa repens, Pygeum africanum* or the composition of the invention on the 5-α-reductase activity in epithelial cells and prostatic fibroblasts, evaluated as a reduction in the amount of converted DHT. Mean ± SD; n = 6
Figure 4 shows the effect of the treatment with the composition of the invention, containing the different components and different extracts of *Serenoa repens,* on the 5α-reductase activity in prostatic epithelial cells evaluated as a reduction in the amount of dihydrotestosterone (DHT) converted. Extract obtained by extraction using a hydroalcoholic mixture with nominal titre *(Serenoa repens)* and by extraction using supercritical CO₂, *(Serenoa repens* SC-CO2). Mean ± SD; (n = 6).

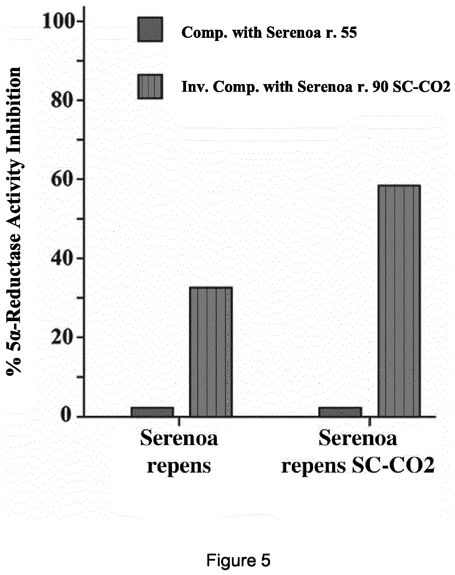

Figure 5 shows the effect of treatment with different types of Serenoa extract on the activity of 5α-reductase type II in prostatic fibroblasts, evaluated as a reduction in the amount of dihydrotestosterone (DHT) converted. Extract obtained by extraction using a hydroalcoholic mixture (*Serenoa repens*) and by extraction using supercritical CO₂ (*Serenoa repens* SC-CO2). Mean ± SD; (n=6).

### DETAILED DESCRIPTION OF THE INVENTION

The invention concerns a composition comprising an extract of *Serenoa repens,* an extract of *Pygeum africanum,* an extract of *Urtica dioica,* an extract of Epilobium and an extract of *Protium heptaphyllum.*

The extracts comprised in the composition of the invention are preferably in the form of powders.

The composition of the invention comprises an extract of *Serenoa repens,* preferably a lipid-sterol extract of *Serenoa repens,* more preferably titrated in the range from about 85 to 95% in fatty acids, even more preferably at 90% in fatty acids. In the composition this extract, involved in the inhibition of 5-α-reductase, contributes to the reduction of prostate development. In addition, the lipid-sterol extract confers anti-inflammatory properties thanks to the modulation of pro-inflammatory genes and the inhibition of cyclooxygenase (COX) and lipoxygenase (5-LOX). Such anti-inflammatory activity resulted to be very important from a symptomatological point of view as the inhibition of COX and 5-LOX enzymes resulted in reduction of edema from inflammatory mediators, unblocking of prostatic urethra and improvement of urinary flow by the composition.

The composition comprises an extract of *Serenoa repens.* Numerous commercial products containing *Serenoa repens* alone or in combination with other phytotherapeutic agents are known and may be used in the composition of the invention. The extract to be used in the composition of the invention has a specific *Serenoa repens* titre and is characterized by a specific extraction method. Preferably, said extraction method is the one using supercritical carbon dioxide.

Advantageously, the extract of *Serenoa repens* obtained with this extraction method is characterized by very high degrees of purity, which affects the composition of the invention. The classical extraction methods, for example those based on hexane involve heating the solution to evaporate the hexane at the end of the process to separate the lipid extract from the toxic hydrocarbon. This process negatively affects the extraction yield; during heating, in fact, part of the lipid extract is altered by heat and lost. Without being bound to any theory, the inventors believe that the method using supercritical carbon dioxide (CO₂), based on the use of carbon dioxide in the liquid/gaseous (supercritical) state, allows to considerably increase the operating pressure and once completed the process, by restoring the atmospheric pressure, to evaporate CO₂ and isolate fatty acids with high degrees of purity, even higher than 90%. In an advantageous and preferred form, therefore, the extract of *Serenoa repens* of the invention is titrated at 90% in fatty acids with supercritical carbon dioxide.

*Serenoa repens,* preferably in the form of an extract titrated at 90% in fatty acids with supercritical carbon dioxide, is present in the composition in an amount from 320 mg to 450 mg, preferably 400 mg, being said amount expressed with respect to a unit dose preparation.

The composition of the invention further comprises a *Pygeum africanum* extract, preferably in the form of a dry extract of *Pygeum africanum* titrated in beta-sitosterols, more preferably titrated from about 1 to 5% in beta-sitosterols, even more preferably at 2.5% in beta-sitosterols. *Pygeum africanum* is an evergreen tree native to equatorial Africa forests. Its bark is known to have been used since 1969 for the treatment of BPH thanks to its anti-inflammatory action. It is in fact known that active compounds of the cortex, such as N,N-butyl benzenesulfonamide and atraric acid, inactivate androgen receptors, inhibit cell growth factors and exert an anti-inflammatory activity. The composition of the invention therefore acts on mediators of inflammation, by inhibiting 5-lipoxygenase, reducing the production and release of leukotrienes and other mediators of inflammation induced by chronic urinary retention. Thanks to the presence of *Pygeum africanum,* the composition of the invention modulates bladder contractility and inhibits the production of fibroblasts in the prostate tissue, acting at the same time on obstructive and irritative symptoms. Unlike other anti-inflammatories such as turmeric, bromelain, boswelia, the composition of the invention comprising pygeum performs its inflammation-reducing action selectively for the prostate tissue. The composition of the invention, thanks mainly to the presence of *Pygeum africanum* having the ability to inhibit aromatase, reduces the risk of gynecomastia linked to the conversion of testosterone into estrogen.

The extract of *Pygeum africanum* is present in the composition of the invention in an amount from 80 to 150 mg, preferably of 100 mg, this amount being expressed with respect to a unit dose preparation.

The composition of the invention also comprises an extract of *Urtica dioica,* preferably in the form of root dry extract titrated in beta-sitosterols as comprising from 0.2 to 1.5% of beta-sitosterols, more preferably about 0.8% of beta-sitosterols.

*Urtica dioica* is an herbaceous plant whose roots are used in the treatment of hypertension. *Urtica dioica* roots contain a complex mixture of substances such as lignans, phenols and sterols which in the composition of the invention allow to inhibit prostate cells proliferation, with a reduction of BPH symptoms. Without being bound to any theory, the inventors believe that *Urtica dioica* acts by inhibiting the proliferation of prostatic cell lines, probably by blocking the EGF receptors and due to a receptor competition of sex hormone binding globulin (SHBG) which regulates the concentration of free androgens and estrogens in plasma; thanks to an interference at the level of prostate cells membrane receptors, the composition comprising *Urtica dioica* prevents hormones from interacting and starting the cell proliferation process. In addition, the extract of *Urtica dioica* has a myorelaxant action on the detrusor muscle responsible for the contraction of the bladder and the bladder sphincter. This particular action makes the composition useful in improving the urinary discharge in case of prostatitis and prostatic hypertrophy. Advantageously, the presence of *Urtica dioica* causes the composition to have an antihypertensive effect due to smooth muscle relaxation by blocking the calcium channels, and this feature differentiates it from alpha-lytic drugs due to the absence of side effects such as hypotension or retrograde ejaculation.

*Urtica dioica* in the form of an extract is present in the composition of the invention in an amount from 80 to 150 mg, preferably of 80 mg, this amount being expressed with respect to a unit dose preparation.

The composition of the invention further comprises an extract of Epilobium, preferably in the form of a dry extract D/E 4:1.

Epilobium, *Epilobium angustifolium,* is a perennial herb that has analgesic, anti-inflammatory, anti-androgenic properties recognized by medicine in the treatment of cancer and prostatic hypertrophy. Hiermann et al. demonstrated the anti-inflammatory effect exerted by the extract of Epilobium, highlighting a significant inhibition in the release of prostaglandins. Analgesic properties comparable to lysine acetylsalicylate were also highlighted In addition to the anti-inflammatory properties. Several species of Epilobium have been shown to have antibacterial properties. Thanks to the presence of Epilobium, the composition of the invention also plays an important role in the inhibition of aromatase and 5 alpha reductase enzymes relevant to the development and maintenance of BPH. The chemical composition of epilobium consists of sterols, tannins, flavonoids (myricetin, quercetin, guaiaverin and kaempferol), fatty acids, aromatic acids, vitamins, and glucosides. Studies in the literature have shown the inhibition of aromatase and 5alpha-reductase with enzymatic tests. The presence of epilobium in the composition causes it to perform an antibiotic-like action, *i.e.* it is capable of reducing the microbial load of the bacterial species most involved in bacterial infections, namely E. coli, Klebsiella, Proteus.

The extract of Epilobium is present in an amount from 30 to 100 mg, preferably 50 mg, this amount being expressed with respect to a unit dose preparation.

The composition of the invention further comprises an extract of *Protium heptaphyllum,* preferably in the form of an extract titrated from about 1 to 5% in α and β amirin, even more preferably a dry extract titrated at 2.5% in α and β amirin.

*Protium heptaphyllum* is a plant belonging to the Burseraceae family. It produces an amorphous resin which has α and β amirin as its main constituents. Protium amirins have shown pharmacological activities in various areas, such as the central and peripheral nervous system, the intestinal tract, and the immune system. In particular, the scientific world is interested in the analgesic and anti-inflammatory properties of Protium and its possible use in the treatment of diseases involving symptoms such as pain, inflammation and edema. Amirins have shown peripheral and central analgesic effects independent of the opioid system in various studies, and also showed potent anti-inflammatory activity. The inventors of the present invention surprisingly found that Protium could act synergistically on the remaining constituents of the composition. The synergistic use of Protium in the composition of the invention has improved the therapy compliance thanks to the synergy generated on the anti-inflammatory and pain-relieving action of the composition.

The extract of *Protium heptaphyllum* is preferably present in an amount from 20 to 100 mg, more preferably of 50 mg, being said amount expressed with respect to a unit dose preparation.

The composition of the invention may further comprise lycopene, preferably in the form of a tomato extract titrated from 10 to 25% in lycopene, about 18% of lycopene. More preferably, the lycopene extract is in an amount from 18.5 to 50 mg.

Lycopene is a carotenoid responsible for the red color of many fruits and vegetables. It is particularly abundant in tomatoes and derivatives thereof, but is also present in watermelon, pink grapefruit and apricots. In the body it can exert a powerful antioxidant action that can protect cells from oxidative damage. Thanks to the presence of lycopene, the composition of the invention has an antioxidant action that protects prostate cells from free radicals released as a result of inflammation.

The composition of the invention may also include additional ingredients that improve its activity.

Among these, zinc can be mentioned. Zinc is an indispensable metal for our body as it is a constituent element of over two hundred enzymes and many other proteins. In particular, it is essential for the functioning of enzymes that regulate cellular respiration, those having an antioxidant action, and some proteins that enable unravelling of tightly entangled DNA in the chromosomes and then read the instructions. Zinc also fights against the negative effects of free radicals and cellular aging processes associated with them. Its presence in the composition of the invention therefore allows to manage and control the oxidative stress generated by the infectious and inflammatory phenomena to which the prostate is subjected. The damage of free radicals can lead to alterations in the cytological stability of prostate cells, resulting in abnormalities in control mechanisms such as cell apoptosis. Zinc may be present in an amount from 5 to 12.5 mg, said amount being expressed with respect to a unit dose preparation.

The composition of the invention may also comprise Vitamin E in an amount from 5 to 50 mg, preferably of 10 mg, being said amount expressed with respect to a unit dose preparation.

There are various forms of vitamin E in nature: four tocopherols and four tocotrienols; Vitamin E is a powerful antioxidant belonging to the category of fat-soluble vitamins so, unlike Vitamin C, it is soluble in fats. It also protects cell membranes from oxidation. As for the antioxidant action of vitamin E, it has the ability to block the chain of radical reactions, which would form highly unstable peroxidic radicals.

Its presence in the composition of the invention therefore allows it to synergistically contribute to the antioxidant action of zinc and selenium, maintain the cytological stability of prostate cells and reduce the damage by free radicals.

The composition of the invention may also advantageously comprise selenium, more preferably in an amount from 20 to 42 mcg.

Selenium is a cofactor of the antioxidant enzymes glutathione peroxidase (GSH-Px) and thioredoxin (Trx) disulfide reductase. By operating in synergy with vitamin E (tocopherol or tocotrienol), the glutathione-peroxidase family is responsible for fighting against oxidative stress from free radicals, especially oxygen reactive species such as hydrogen peroxide and organic hydroperoxides, on cell membranes. Selenium is an essential micronutrient, which means that the body is unable to produce it autonomously and that in case of insufficient intake there is a deficiency that predisposes tissues, which are vulnerable to inflammation, to oxidative damage from free radicals. Since the prostate is a vulnerable tissue, the composition of the invention benefits from an anti-inflammatory effect.

The composition of the invention has therefore resulted in a selected and synergistic combination of active ingredients capable of treating and preventing benign prostatic hyperplasia.

According to a primary aspect of the invention, the invention concerns the use of the composition comprising the composition of the invention for use as a medicament.

According to a further aspect, the invention concerns the composition described above for use in the treatment of benign prostatic hyperplasia.

The invention also concerns a supplement comprising the composition of the invention and excipients for use in the treatment of benign prostatic hyperplasia.

The composition and supplement for use in the treatment of benign prostatic hyperplasia may preferably be administered by oral administration.

The compositions may therefore be in liquid, solid or semi-solid form, and may be in the form of tablets, pearls, drops, sticks, syrups, sachets.

The compositions of the invention may be administered orally in the form of tablets, capsules, granules, effervescent granules, syrups or the like.

They can also be dissolvable in water to form inhalable solutions.

The compositions in solid form may comprise one or more additives such as starches, sugars, cellulose, diluents, granulating agents, lubricants, binders, disintegrating agents, stabilizers, emulsifiers, texturizers, plasticizers, wetting agents, thickeners, coatings. All these excipients allow to obtain the supplement of the invention in the desired pharmaceutical formulation.

The capsules may be coated with a gastro-resistant film, or they may be soft gel capsules.

Flavoring agents, preservatives, colouring agents or the like may also be present.

The active ingredients contained in the compositions object of the present invention may be combined or mixed as known active ingredients and/or excipients according to pharmaceutical techniques and techniques of the food or nutritional industry, and prepared by methods known in the pharmaceutical or food industry.

The compositions object of the invention are also used as adjuvants in the treatment of benign prostatic hyperplasia (IPB).

In an advantageous aspect of the invention, the composition of the invention may be combined with at least one 5-α reductase enzyme inhibitor drug at least one α-lytic drug.

The composition of the invention may, in fact, be administered alone or in combination, *i.e.* as a co-therapeutic agent in a fixed-dose combination or in a combined therapy of separately formulated active ingredients, with at least one 5-α reductase enzyme inhibitor drug, such as for example Finasteride, Dutasteride, and/or at least one alpha-lytic drug, such as for example Doxazosin, Prazosin, Alfuzosin, Terazosin, Tamsulosin, Silodosin, for further potentiation and faster resolution of lower urinary tract symptoms (LUTS).

In fact, the composition of the invention, *i.e.* the medicament or the supplement comprising the same, has an excellent anti-microbial, anti-inflammatory and anti-androgenic activity. Specifically, the product of the invention showed excellent antimicrobial activities against Escherichia coli, anti-inflammatory activity against pro-inflammatory cytokines and/or chemokines IL-6, TNFα, and antiandrogenic activity, thanks to the ability to inhibit 5-α-reductase evaluated as a reduction in the amount of Dihydrotestosterone (DHT).

In particular, the composition of the invention is therefore useful as a dietary and nutritional supplement, as a medicament for the prevention and treatment of benign prostatic hyperplasia.

The compositions object of the invention are therefore pharmaceutical preparations, nutraceuticals preparation or food supplements that can be introduced in the dietary regime of an individual suffering from benign prostatic hyperplasia or presenting one or more of its symptoms.

The composition object of the present invention is therefore a valid therapeutic tool for the management of benign prostatic hyperplasia symptoms.

In this sense, the composition of the invention also has a preventive action, since it avoids the increase in the number of prostate cells (prostatic hyperplasia) thanks to inhibition of 5-α-reductase enzyme. In addition, the anti-inflammatory action decreases the risk of an increase in the volume of prostate cells due to edema (prostatic hypertrophy).

The synergy generated by the combination above showed its effectiveness in enhancing the effects of its components which was greater than the sum of the known effects of the individual active ingredients.

### EXPERIMENTAL PART

### Example 1: Preparation of the composition of the invention

For the preparation of the composition of the invention and therefore of the final product, such as for example a supplement, the following were used:
- An extract of *Serenoa repens* titrated at 90% in fatty acids in supercritical CO₂. It was present in the composition in an amount from 320 mg to 450 mg, preferably of 400 mg;
- A dry extract of *Pygeum africanum* titrated at 2.5% in beta-sitosterols. It was present in an amount from 80 to 150 mg, preferably of 100 mg.
- A dry extract of *Urtica dioica* comprising about 0.8% of β-sitosterols. *Urtica dioica* was present in an amount from 80 to 150 mg, preferably of 80 mg;
- An extract of *Epilobium angustifolium,* dry extract D/E 4:1. It was present in an amount from 30 to 100 mg, preferably of 50 mg.
- A *Protium heptaphyllum* extract The extract of *Protium heptaphyllum,* preferably in the form of a dry extract titrated at 2.5% in α and β amirin.

The above composition could further comprise:
- Lycopene, *Solanum lycopersicum,* in the form of a tomato extract titrated at 18% lycopene. When present, it was present in an amount from 18.5 to 50 mg;
- Zinc present in an amount from 5 to 12.5 mg, and
- Selenium present in an amount from 20 to 42 mcg
- Vitamin E present in an amount from 5 to 10 mg

All amounts were expressed with respect to unit dose preparations.

The compositions of the invention could be formed by combining the above ingredients in the amounts indicated, preferably the preferred ones.

### Example 2: Evaluation of the composition of the invention.

The composition of the invention was tested through an *in vitro* research study carried out at the Pharmacy Department of the University of Salerno.

The composition of the invention was prepared using a geometric dilution method so as to obtain a homogeneous mixing of the powders in a mortar. The mixture is prepared following the ratios:
- 400 mg of *Serenoa repens* titrated at 90% in fat acids extracted in supercritical CO₂
- 100 mg of *Pygeum africanum*
- 80 mg of *Urtica dioica*
- 50 mg of *Protium heptaphyllum*
- 50 mg of *Epilobium angustifolium.*

The amounts of the above ingredients were expressed with respect to a unit dose preparation.

Evaluations were made in order to demonstrate the ability of the product to be used in the treatment of benign prostatic hyperplasia.

### Antibacterial activity

The first evaluation concerned the ability of the composition of the invention to inhibit bacterial growth.

For determining the bacterial growth inhibition capacity of the composition of the invention, a gram-negative bacterium typically related to urinary tract and prostate infections was used, that is Escherichia coli.

The antimicrobial efficacy of the composition over time was assessed by means of a "time-killing assay" carried out against Escherichia coli in co-culture with VCaP cells (immortalized prostate cells). The bacterial colonies were inoculated into wells containing the cell cultures and after one night they were treated with saline solution as a control, with a solution containing extract of epilobium or with the composition of the invention. After incubation, live bacterial colonies were calculated at different time intervals (1,3, and 7 days). The experiments were carried out in triplicate for each single experimental point.

Figure 1 shows the results, specifically the number of colony forming units (CFU) related to Escherichia coli, after exposure of bacteria in co-culture with previously contaminated VCaP cells with appropriate concentrations of microorganisms, treated with control solution, treated with a solution containing an extract of *Epilobium angustifolium,* and a solution containing the composition of the invention in the proportions indicated. The results demonstrated how an aqueous solution of Epilobium extract is able to inhibit the proliferation of E. coli at reduced concentrations with a substantial reduction in CFUs to values close to 0.5 mg/mL. In fact, as shown in Figure 1, the treatment of the bacterial culture with a solution of Epilobium extract showed a statistically significant increase in the inhibition of bacterial growth in the conditions tested for up to 7 days. It should also be emphasized that the composition of the invention containing the same amount of Epilobium together with the other extracts indicated, was able to further reduce the proliferative activity of E. Coli by about 40%, compared to the Epilobium extract alone, both at 1 and 3 days.

### Anti-inflammatory activity

The second evaluation was related to the anti-inflammatory activity of the composition of the invention. Studies aimed at verifying the anti-inflammatory activity of the composition of the invention were carried out by means of an ELISA assay of pro-inflammatory cytokines and/or chemokines: IL-6, TNFα. The ELISA assay involved the use of two antibodies and a detection system to identify the aforementioned cytokines in immunosorbent plates after spectrophotometric reading (wavelength 450 nm, corrected to 550 nm). In particular, VCaP cells were treated with the vehicle, the control and the composition of the invention. The cellular treatment lasted for 24 hours in order to correlate the results obtained for cell viability with the release of pro-inflammatory cytokines.

VCaP cells were then treated with increasing concentrations of the single *Protium heptaphyllum* extract, as well as with the whole composition containing all the extracts. In the concentrations indicated there were no alterations in cell viability, and no solid residues were found in the culture medium visible under an optical microscope. As can be seen in Figure 2A, the different extracts are able to statistically significantly reduce the release of IL6 and TNFα at all tested concentrations. Furthermore, the composition of the invention was able to further reduce the release of cytokines compared to single extracts.

VCaP cells (immortalized prostate cells) were also treated with a mixture made of *Serenoa repens* SC-CO2, *Pygeum africanum, Urtica dioica* and *Epilobium augustifolium,* as well as with the Formulation object of the invention containing the same amount of the respective extracts, and with the extract of *Protium heptaphyllum* alone, because these concentrations, as previously demonstrated, are soluble in the culture medium, and do not alter cell viability.

As can be seen from Figure 2B, the Formulation of the invention was able to reduce the release of IL6 interleukins by over 200% with respect to *Protium heptaphyllum* extract and by over 20% compared to the mixture of extracts containing *Serenoa repens* SC-CO2, *Pygeum africanum, Urtica dioica* and *Epilobium augustifolium* at all tested concentrations. Furthermore, the composition of the invention was able to reduce the release of TNF-α by more than 60% with respect to *Protium heptaphyllum* extract and by more than 20% compared to the mixture made of *Serenoa repens* SC-CO2, *Pygeum africanum, Urtica dioica* and *Epilobium augustifolium* extracts at all tested concentrations.

Surprisingly, the whole composition of the invention showed an increase in anti-inflammatory activity of a further 20% compared to the mixture of *Serenoa, Pygeum, Urtica* and *Epilobium.* This result showed that *Protium heptaphyllum* contributed synergistically to the reduction of IL-6 and TNF-α, when used together with the other extracts in the composition of the invention.

### Antiandrogenic activity

The composition of the invention was also evaluated to verify its anti-androgenic activity. For this purpose, studies were carried out on co-cultured prostate cells (10 days) by mixing epithelial cells with prostatic fibroblasts in equal parts. The cells, after being harvested, were suspended in addition to testosterone, and incubated for 30 minutes. After treatment, the cells were resuspended in ethanol and the 5α reductase activity was measured based on the conversion of testosterone into dihydrotestosterone (DHT). The enzymatic activity was then expressed as a percentage with respect to the control.

The antiandrogenic activity of the composition of the invention, of Serenoa and Pygeum, was tested on prostate cells by verifying the ability to inhibit 5-α-reductase evaluated as a reduction in the amount of Dihydrotestosterone (DHT). As can be seen in Figure 3, both the extract of *Serenoa repens* and the extract of *Pygeum africanum* are able to significantly reduce the activity of 5-α-reductase. Furthermore, the composition of the invention proved to be more effective than the individual extracts. Specifically, the mixture was more effective than the already substantial inhibition action seen above, reaching and overcoming an 80% reduction in enzymatic activity. This is thanks to the synergy of action triggered by the active ingredients of *Serenoa repens* and Pygeum which, to a significantly greater extent, are able to specifically inhibit the 5α-reductase enzyme responsible for prostatic growth.

### Antiandrogenic activity

The antiandrogenic activity of the composition of the invention was tested on prostate cells by verifying the ability to inhibit 5α-reductase evaluated as a reduction in the amount of dihydrotestosterone (DHT) converted. In addition, various extracts of *Serenoa repens* were used, coming from:
- Extraction method using supercritical CO₂ with a nominal content of 90% fatty acids
- Extraction method with water and ethanol with a nominal titre of 55% fatty acids These extracts are tested in "raw" form and also tested in mixture with the other components of the composition of the invention, in the same proportions.

As can be noted in Figure 4 and Figure 5, among the various extracts of *Serenoa repens,* the one obtained by extraction using supercritical CO₂ (90% nominal titre) has always proved to be more effective than that obtained from hydroalcoholic extraction. This effect is also apparent when the extract is in a mixture. In fact, the composition of the invention containing the extract of *Serenoa repens* coming from extraction with supercritical CO₂ shows an efficiency greater by about 15% in inhibiting the 5α-reductase with respect to the mixture containing the extract of Serenoa obtained from hydroalcoholic extraction. Furthermore, the composition of the invention proved to be more effective than the individual extracts when tested against 5α-reductase.

## Claims

1. A composition comprising an extract of *Serenoa repens,* an extract of *Pygeum africanum,* an extract of *Urtica dioica,* an extract of Epilobium and an extract of *Protium heptaphyllum.*

2. The composition of claim 1, wherein the extract of *Serenoa repens* is a lipid-sterol extract of *Serenoa repens,* preferably obtained by an extraction method with supercritical carbon dioxide.

3. The composition according to claim 1 or 2, wherein the extract of *Serenoa repens* is titrated in the range from about 85 to 95% in fatty acids, even more preferably at 90% in fatty acids.

4. The composition according to anyone of claims 1 to 3, wherein the extract of *Serenoa repens* is present in an amount from 320 mg to 450 mg, preferably of 400 mg, being said amount expressed with respect to a unit dose preparation.

5. The composition according to anyone of claims 1 to 4, wherein the *Pygeum africanum* extract is present in the composition of the invention in an amount from 80 to 150 mg, preferably of 100 mg, being said amount expressed with respect to a unit dose preparation.

6. The composition according to anyone of claims 1 to 5, wherein the extract of *Urtica dioica* is present in an amount from 80 to 150 mg, preferably of 80 mg, being said amount expressed with respect to a unit dose preparation, more preferably in the form of a root dry extract comprising from 0.2 to 5%, still more preferably about 0.8%, of β-sitosterols.

7. The composition according to anyone of claims from 1 to 6, wherein the extract of Epilobium is present in an amount from 30 to 100 mg, preferably of 50 mg, being said amount expressed with respect to a unit dose preparation, more preferably in the form of a dry extract D/E 4:1.

8. The composition according to anyone of claims 1 to 7, wherein the extract of *Protium heptaphyllum* is present in an amount from 20 to 100 mg, being said amount expressed with respect to a unit dose preparation, preferably in the form of an extract titrated from about 1 to 5% in α and β amirin, more preferably dry titrated at 2.5% in α and β amirin.

9. The composition according to anyone of claims 1 to 8, wherein said composition further comprises at least one ingredient selected from lycopene, preferably in an amount from 18.5 to 50 mg, zinc, preferably in an amount from 5 to 12.5 mg, Vitamin E, and selenium, more preferably in an amount from 20 to 42 µg, being said amounts expressed with respect to a unit dose preparation.

10. A composition according to anyone of claims 1 to 9 for use as a medicament.

11. A supplement comprising the composition according to anyone of claims 1 to 9 and excipients.

12. A composition according to anyone of claims 1 to 9 for use in the treatment and in the prevention of benign prostatic hyperplasia or the supplement of claim 11 for use in the treatment and in the prevention of benign prostatic hyperplasia.

13. The composition for use according to claim 12, wherein the composition is administered in combination, that is as a co-therapeutic agent, in a fixed-dose combination or in a combined therapy of separately formulated active ingredients, with at least one 5-α reductase enzyme inhibitor drug and/or at least one α-lytic drug for further potentiation and faster resolution of lower urinary tract symptoms (LUTS).

## Patentansprüche

1. Zusammensetzung, die einen Extrakt von Serenoa repens, einen Extrakt von Pygeum africanum, einen Extrakt von Urtica dioica, einen Extrakt von Epilobium und einen Extrakt von Protium heptaphyllum aufweist.

2. Zusammensetzung nach Anspruch 1, wobei der Extrakt von Serenoa repens ein Lipid-Sterol-Extrakt von Serenoa repens ist, der vorzugsweise durch ein Extraktionsverfahren mit überkritischem Kohlendioxid erhalten wird.

3. Zusammensetzung nach Anspruch 1 oder 2, wobei der Extrakt aus Serenoa repens im Bereich von etwa 85 bis 95 % Fettsäuren, vorzugsweise sogar bis zu 90 % aus Fettsäuren besteht.

4. Zusammensetzung nach einem der Ansprüche 1 bis 3, wobei der Extrakt aus Serenoa repens in einer Menge von 320 mg - 450 mg, vorzugsweise von 400 mg, vorliegt, wobei die Menge in Bezug auf eine Einheitsdosis-Zubereitung ausgedrückt ist.

5. Zusammensetzung nach einem der Ansprüche 1 bis 4, wobei der Pygeum africanum-Extrakt in der erfindungsgemäßen Zusammensetzung in einer Menge von 80 bis 150 mg, vorzugsweise von 100 mg, vorhanden ist, wobei die Menge in Bezug auf eine Einheitsdosis-Zubereitung ausgedrückt ist.

6. Zusammensetzung nach einem der Ansprüche 1 bis 5, wobei der Extrakt von Urtica dioica in einer Menge von 80 bis 150 mg, vorzugsweise 80 mg, vorliegt, wobei die Menge in Bezug auf eine Einheitsdosis-Zubereitung ausgedrückt ist, vorzugsweise in Form eines Wurzel-Trockenextrakts, der 0,2 bis 5 %, noch bevorzugter etwa 0,8 %, β-Sitosterole aufweist.

7. Zusammensetzung nach einem der Ansprüche 1 bis 6, wobei der Epilobium-Extrakt in einer Menge von 30 bis 100 mg, vorzugsweise von 50 mg, vorliegt, wobei die Menge in Bezug auf eine Einheitsdosis-Zubereitung ausgedrückt ist, vorzugsweise in Form eines Trockenextrakts D/E 4:1.

8. Zusammensetzung nach einem der Ansprüche 1 bis 7, **dadurch gekennzeichnet, daß** der Extrakt von Protium heptaphyllum in einer Menge von 20 bis 100 mg vorliegt, wobei die Menge auf eine Einheitsdosis-Zubereitung ausgedrückt ist, vorzugsweise in Form eines Extraktes, der mit etwa 1 bis 5 % in a und β-Amirin titriert ist, besonders bevorzugt trocken mit 2,5 % in a und β-Amirin titriert ist.

9. Zusammensetzung nach einem der Ansprüche 1 bis 8, wobei die Zusammensetzung weiterhin mindestens einen Bestandteil aufweist, der aus Lycopin, vorzugsweise in einer Menge von 18,5 bis 50 mg, Zink, vorzugsweise in einer Menge von 5 bis 12,5 mg, Vitamin E und Selen, besonders bevorzugt in einer Menge von 20 bis 42 µg, ausgewählt ist, wobei die Mengen in Bezug auf eine Einheitsdosiszubereitung ausgedrückt sind.

10. Zusammensetzung nach einem der Ansprüche 1 bis 9 zur Verwendung als Arzneimittel.

11. Nahrungsergänzungsmittel, das die Zusammensetzung nach einem der Ansprüche 1 bis 9 und Hilfsstoffe aufweist.

12. Zusammensetzung nach einem der Ansprüche 1 bis 9 zur Verwendung bei der Behandlung und bei der Vorbeugung der gutartigen Prostatahyperplasie oder das Ergänzungsmittel nach Anspruch 11 zur Verwendung bei der Behandlung und bei der Vorbeugung der gutartigen Prostatahyperplasie.

13. Zusammensetzung zur Verwendung nach Anspruch 12, wobei die Zusammensetzung in Kombination, d.h. als Co-Therapeutikum, in einer Festdosiskombination oder in einer Kombinationstherapie von getrennt formulierten Wirkstoffen, mit mindestens einem 5-a-Reduktase-Enzym-Inhibitor-Medikament und/oder mindestens einem a-lytischen Medikament zur weiteren Potenzierung und schnelleren Auflösung von Symptomen des unteren Harntrakts (LUTS) verabreicht wird.

## Revendications

1. Composition comprenant un extrait de *Serenoa repens,* un extrait de *Pygeum africanum,* un extrait *d'Urtica dioica,* un extrait d'épilobe et un extrait de *Protium heptaphyllum.*

2. Composition de la revendication 1, dans laquelle l'extrait de *Serenoa repens* est un extrait lipide-stérol de *Serenoa repens,* préférentiellement obtenu par une méthode d'extraction au dioxyde de carbone supercritique.

3. Composition selon la revendication 1 ou 2, dans laquelle l'extrait de *Serenoa repens* est titré dans la gamme d'environ 85 à 95 % en acides gras, encore plus préférentiellement à 90 % en acides gras.

4. Composition selon l'une quelconque des revendications 1 à 3, dans laquelle l'extrait de *Serenoa repens* est présent dans une quantité de 320 mg à 450 mg, préférentiellement de 400 mg, ladite quantité étant exprimée par rapport à une préparation à dose unitaire.

5. Composition selon l'une des revendications 1 à 4, dans laquelle l'extrait de *Pygeum africanum* est présent dans la composition de l'invention dans une quantité de 80 à 150 mg, préférentiellement de 100 mg, ladite quantité étant exprimée par rapport à une préparation à dose unitaire.

6. Composition selon l'une quelconque des revendications 1 à 5, dans laquelle l'extrait d*'Urtica dioica* est présent dans une quantité de 80 à 150 mg, préférentiellement de 80 mg, ladite quantité étant exprimée par rapport à une préparation à dose unitaire, plus préférentiellement sous la forme d'un extrait sec de racine comprenant de 0,2 à 5 %, encore plus préférentiellement environ 0,8 %, de β-sitostérols.

7. Composition selon l'une quelconque des revendications 1 à 6, dans laquelle l'extrait d'épilobe est présent dans une quantité de 30 à 100 mg, préférentiellement de 50 mg, ladite quantité étant exprimée par rapport à une préparation à dose unitaire, plus préférentiellement sous la forme d'un extrait sec D/E 4:1.

8. Composition selon l'une quelconque des revendications 1 à 7, dans laquelle l'extrait de *Protium heptaphyllum* est présent dans une quantité de 20 à 100 mg, ladite quantité étant exprimée par rapport à une préparation à dose unitaire, préférentiellement sous la forme d'un extrait titré d'environ 1 à 5 % en α et β amirine, plus préférentiellement titré à sec à 2,5 % en α et β amirine.

9. Composition selon l'une quelconque des revendications 1 à 8, dans laquelle ladite composition comprend en outre au moins un ingrédient choisi parmi le lycopène, préférentiellement dans une quantité de 18,5 à 50 mg, le zinc, préférentiellement dans une quantité de 5 à 12,5 mg, la vitamine E et le sélénium, plus préférentiellement dans une quantité de 20 à 42 µg, ces quantités étant exprimées par rapport à une préparation à dose unitaire.

10. Composition selon l'une quelconque des revendications 1 à 9 pour une utilisation comme médicament.

11. Complément comprenant la composition selon l'une quelconque des revendications 1 à 9 et des excipients.

12. Composition selon l'une quelconque des revendications 1 à 9 pour une utilisation dans le traitement et la prévention de l'hyperplasie bénigne de la prostate ou supplément selon la revendication 11 pour une utilisation dans le traitement et la prévention de l'hyperplasie bénigne de la prostate.

13. Composition pour une utilisation selon la revendication 12, dans laquelle la composition est administrée en association, c'est-à-dire en tant qu'agent co-thérapeutique, dans une association à dose fixe ou dans une thérapie combinée de principes actifs formulés séparément, avec au moins un médicament inhibiteur de l'enzyme 5-a-réductase et/ou au moins un médicament α-lytique pour une potentialisation supplémentaire et une résolution plus rapide des symptômes des voies urinaires inférieures (LUTS).
